# EUROPÄISCHE PATENTSCHRIFT

(11) **EP 1 269 934 B1**
(45) Veröffentlichungstag und Bekanntmachung des Hinweises auf die Patenterteilung: **06.06.2007**
(21) Anmeldenummer: 02011137.3
(22) Anmeldetag: 21.05.2002
(51) Int. Cl.: A61C 13/00

(54) **Verfahren zur Herstellung einer dentalen Prothese**
Method for producing a dental prosthesis
Méthode de fabrication d'une prothèse dentaire

(30) Priorität: 28.06.2001 DE 10131131
(43) Veröffentlichungstag der Anmeldung: 02.01.2003
(73) Patentinhaber: BEGO Bremer Goldschlägerei Wilh. Herbst GmbH & Co. KG, 28359 Bremen (DE)
(72) Erfinder: Strietzel, Roland, Dr., 28865 Lilienthal (DE)
(74) Vertreter: Eisenführ, Speiser & Partner

(56) Entgegenhaltungen:
- EP-A- 0 565 175
- US-A- 4 611 288
- US-A- 5 092 022

## Beschreibung

Die Erfindung betrifft ein Verfahren zur Herstellung einer dentalen Prothese als (Teil-)Ersatz einzelner Zähne oder Zahngruppen im Zahnverbund eines Kiefers unter intraoraler Aufnahme der räumlichen Informationen des die Prothese aufnehmenden Zahnstumpfes, Implantats o. dgl.

Es ist bekannt, die räumliche Konfiguration eines im Kiefer verankerten Trägers für eine Zahnprothese abzutasten, aufzuzeichnen und zu speichern, um mit Hilfe der so gewonnenen Informationen (Daten) die dem Träger anzupassende räumliche Innenkontur bei der Herstellung der Zahnprothese zu konfigurieren (via DE 198 28 003 A1). Letzteres gilt beispielsweise für Kronen und Käppchen sowie Onlays, während bei einer Zahnprothese in Form eines Inlays Außen- und Innenkontur vertauscht sind.

Bei dem Träger kann es sich um einen natürlichen Zahnstumpf handeln, aber auch um ein zuvor im Kiefer verankertes, insbesondere zwischenzeitlich eingewachsenes Implantat. Die intraorale Aufnahme der räumlichen Trägerkonfiguration erfolgt heute im Allgemeinen durch Scannen und Anfertigung einer Datei digitaler Daten. Das Abtasten und Aufnehmen der Träger-Raumform kann jedoch auch durch andere Mittel, beispielsweise Ultraschall, Röntgen oder Computertomografie erfolgen.

Für die Herstellung von Onlays oder Inlays reicht die Kenntnis der so ermittelten "Anschlussdaten" aus. Das ist jedoch nicht der Fall, wenn zur Herstellung von Käppchen, Kronen oder Brücken die Kontaktpunkte bzw. -flächen zu den Nachbarzähnen bei der Gestaltung der Außenflächen derartiger Prothesen festgelegt werden müssen. Bei der Konstruktion von Brücken und/oder mehreren Kronen müssen zusätzlich Informationen über den Gegenkiefer vorliegen, um die Okklusion zu berücksichtigen. Die Bestimmung der Kontaktpunkte und der Okklusion (statisch und dynamisch) erfordert hohe Präzision im Bereich von ca. 10µm.

Im Stand der Technik werden daher, soweit insbesondere Kronen oder Brücken hergestellt werden sollen, nach wie vor mechanische Abdrucke des Kiefers oder der Kiefer bzw. der betreffenden Kieferbereiche abgenommen, also Abformungen, welche Negativformen für die erforderliche Herstellung von Kiefermodellen bilden. Das dauert beträchtliche Zeit (einschließlich Vor- und Nachbearbeitung etwa ¼ Stunde pro Kiefer) und belastet den Patienten entsprechend. Der Abdruck wird nach Desinfizierung verpackt und in das Dentallabor geschickt, wo es der Modellherstellung durch einen Zahntechniker dient. Auf diesem bzw. diesen wird alsdann die gewünschte Prothese angefertigt. Es ist auch bekannt, die mit Hilfe konventioneller Abformungen hergestellten Kiefermodelle zu digitalisieren, d.h. ihre Raumform und räumliche Zuordnung digital aufzunehmen und in einer Datei zu speichern (WO 01/41670).

Aus US 4,611,288 ist ein Verfahren zur Herstellung dentaler Prothesen bekannt, bei dem die Daten des zu versorgenden Zahnes/Zahnstumpfes optisch erfasst werden, die nach Implantation außenliegenden Flächen der Prothese aus vorherbestimmten Standarddaten ausgewählt werden oder aus zusätzlich erfassten Daten des Gebisses berechnet werden und auf Basis dieser Daten die Prothese automatisch hergestellt wird.

Die Erfindung will diese Prozedur nachhaltig vereinfachen. Sie besteht darin, dass die intraorale Aufnahme der Informationen auch auf die Nachbarzähne der herzustellenden Prothese und mindestens den davon betroffenen Bereich des Kiefers erstreckt wird, dass mit Hilfe dieser Informationen ein Modell des Kiefers bzw. Kieferabschnitts mit den Nachbarzähnen angefertigt wird, dass unabhängig davon aus einem Teil der Informationen ein dem Zahnstumpf o. dgl. angepasstes Gerüst für die Prothese hergestellt und danach die Prothese auf dem Gerüst unter Anpassung an die Nachbarzähne angefertigt wird.

Auf diese Weise lässt sich im Rahmen eines einzigen (intraoralen) Scan-Vorganges nicht nur eine speicherbare Aufnahme der dem Träger angepassten Innenkonfiguration des anzufertigenden Zahnersatzes gewinnen, sondern quasi gleichzeitig auch eine Datei der räumlichen Außenkonfiguration der angrenzenden Zähne und ggf. der des Gegenkiefers. Für den Patienten dauert dieser Vorgang einen Bruchteil der für die konventionelle Abdrucknahme erforderlichen Zeit. Die intraoral gescannte Aufnahme kann, ohne dass zusätzliche Bearbeitungen erforderlich wären, einem Zahnlabor zugeleitet werden, und zwar am einfachsten und schnellsten mit Mitteln der Telekommunikation. Der Empfänger stellt mit Hilfe dieser Daten sowohl ein Modell des betroffenen Kiefers bzw. Kieferbereichs (und ggf. des Gegenkiefers oder -bereichs) als auch die gewünschte Zahnprothese oder zunächst ein Gerüst für diese her. Mit Hilfe der zur Verfügung stehenden Daten (bzw. eines heraus ausgekoppelten Teils) kann bei der Prothesenfertigung auf der Grundlage des Modells und ggf. des Gerüsts die Außenkonfiguration der Prothese bezüglich der Nachbarzähne bzw. des Gegenbisses weitgehend angepasst werden. Am Modell braucht nur noch wenig nachgearbeitet zu werden. Auch die statische und/oder dynamische Okklusion (ggf. durch Berechnung) kann konstruktiv beachtet werden. Entsprechend verringert sich der unmittelbare Herstellungsaufwand für den Zahnersatz und somit dessen Kosten.

Wesentlich ist dabei, dass sowohl die Modell- als auch die Gerüst-Herstellung quasi industriell und somit schnell, genau und kostengünstig erfolgen kann, während dann die Prothese zumindest weitgehend manuell angefertigt wird, so dass der Zahntechniker - nicht zuletzt auf Grund seiner Erfahrung - alle individuellen Umstände berücksichtigen kann. Diese naturgemäß teure Maßnahme bleibt aber auf den Teil des gesamten Herstellungsprozesses beschränkt, wo sie tatsächlich benötigt wird.

Die Anfertigung des Modells und/oder des Gerüsts kann namentlich mit Hilfe des von den Informationen (Daten) gesteuerten "Rapid Prototyping" erfolgen, also beispielsweise durch Fräsen, aber auch einen Schichtaufbau mittels Lasersinterns oder mittels eines 3D-Printers. Das Gerüst wird im allgemeinen aus Metall bestehen, kann aber auch aus Keramik oder Kunst-stoff hergestellt werden, während die Prothese ihre endgültige Form durch eine - manuell aufgebrachte - Verblendung aus Keramik (Porzellan) erhält.

## Patentansprüche

1. Verfahren zur Herstellung einer dentalen Prothese als Teil- oder Vollersatz einzelner Zähne oder Zahngruppen im Zahnverbund eines Kiefers unter intraoraler Aufnahme der räumlichen Informationen des die Prothese aufnehmenden Zahnstumpfes, Implantats o. dgl.,
wobei die intraorale Aufnahme der Informationen auch auf die Nachbarzähne der herzustellenden Prothese und mindestens den davon betroffenen Bereich des Kiefers erstreckt wird,
wobei mit Hilfe dieser Informationen ein Modell des Kiefers bzw. Kieferabschnitts mit den Nachbarzähnen angefertigt wird, unabhängig davon aus einem Teil der Informationen ein dem Zahnstumpf o. dgl. angepasstes Gerüst für die Prothese hergestellt und danach die Prothese auf dem Gerüst unter Anpassung an die Nachbarzähne weitgehend manuell auf der Grundlage des Modells weiter bearbeitet und/oder fertiggestellt wird.

2. Verfahren nach Anspruch 1, **dadurch gekennzeichnet, dass** zusätzlich mindestens der betroffene Bereich des Gegenkiefers aufgenommen und in die Modellherstellung einbezogen wird.

3. Verfahren nach Anspruch 1 oder 2, **dadurch gekennzeichnet, dass** die Informationen des Zahnstumpfes o. dgl. aus den intraoral aufgenommenen Informationen als Teil hiervon ausgekoppelt werden.

4. Verfahren nach einem der vorhergehenden Ansprüche, **dadurch gekennzeichnet, dass** bei der Anfertigung der Prothese die statische und/oder dynamische Okklusion aus den aufgenommenen Daten ermittelt und konstruktiv berücksichtigt wird.

## Claims

1. A method for producing a dental prosthesis as partial or full replacement of individual teeth or groups of teeth in the dental arch of a jaw with intraoral recording of the spatial information of the tooth stump, implant or the like receiving the prosthesis,
the intraoral recording of the information also being extended to the adj acent teeth of the prosthesis to be produced and at least the area of the jaw thereby affected,
a model of the jaw or portion of the jaw with the adjacent teeth being prepared with the aid of this information, independently thereof a support structure for the prosthesis, adapted to the tooth stump or the like, being produced from a part of the information and thereafter the prosthesis being further processed and/or completed on the support structure with adaptation to the adjacent teeth largely manually on the basis of the model.

2. A method according to Claim 1, **characterised in that** in addition at least the affected area of the opposite jaw is recorded and incorporated into the production of the model.

3. A method according to Claim 1 or 2, **characterised in that** the information of the tooth stump or the like is extracted from the intraorally recorded information as part thereof.

4. A method according to one of the preceding claims, **characterised in that** in the preparation of the prosthesis the static and/or dynamic occlusion is determined from the recorded data and structurally taken into consideration.

## Revendications

1. Procédé de fabrication d'une prothèse dentaire en tant que substitut partiel ou intégral de différentes dents ou groupes de dents dans l'arcade dentaire d'une mâchoire par saisie intraorale des informations spatiales du moignon dentaire recevant la prothèse, de l'implant ou similaire,
où la saisie intraorale des informations s'étend aussi aux dents contiguës à la prothèse à fabriquer et au moins à la zone concernée de la mâchoire,
où un modèle de la mâchoire ou de la partie de mâchoire avec les dents contiguës est réalisé au moyen de ces informations, où indépendamment une structure adaptée au moignon dentaire ou similaire est réalisée pour la prothèse sur la base d'une partie des informations, et où la prothèse est ensuite ajustée et/ou terminée sur la structure en étant adaptée aux dents contiguës de manière essentiellement manuelle, sur la base du modèle.

2. Procédé selon la revendication 1, **caractérisé en ce qu'**au moins la zone concernée de la mâchoire opposée est en outre saisie et intégrée à la réalisation de modèle.

3. Procédé selon la revendication 1 ou la revendication 2, **caractérisé en ce que** les informations du moignon dentaire ou similaire sont désassemblées des informations saisies intraoralement en tant que partie de celles-ci.

4. Procédé selon l'une des revendications précédentes, **caractérisé en ce que**, lors de la fabrication de la prothèse, l'occlusion statique et/ou dynamique est déterminée à partir des données saisies et prise constructivement en compte.
